# EUROPEAN PATENT SPECIFICATION

(11) **EP 3 138 487 B1**
(45) Date of publication and mention of the grant of the patent: **04.10.2023**
(21) Application number: 16183023.7
(22) Date of filing: 05.08.2016
(51) Int. Cl.: A61B 5/097, A61B 5/08, G01N 33/497, A24D 3/18, A24F 7/00, A24F 13/06

(54) **MOUTHPIECE**
MUNDSTÜCK
EMBOUCHURE

(30) Priority: 14.08.2015 GB 201514491
(43) Date of publication of application: 08.03.2017
(73) Proprietor: Quirke, Daniel, Otham, Kent ME15 8RJ (GB)
(72) Inventor: Quirke, Daniel, Otham, Kent ME15 8RJ (GB)
(74) Representative: Bridle, Andrew Barry

(56) References cited:
- EP-A1- 2 098 166
- EP-A2- 2 745 776
- WO-A1-01/39685
- WO-A1-01/84112
- WO-A1-90/14122
- WO-A2-2011/143289
- WO-A2-2012/068374
- FR-A1- 2 374 919
- US-A- 5 394 867

## Description

The present invention relates to mouthpieces for devices, such as diagnostic or therapeutic respiratory devices. In particular it relates to filtered mouthpieces.

Many devices have been designed to analyse certain features relating to a subject from a breath sample. Such devices include carbon monoxide (CO) detectors which are used to detect levels of carbon monoxide in the exhaled gases of a subject, hydrogen breath detectors which are similar to CO detectors, but which measure levels of hydrogen in the exhaled gases, and alcohol detectors which measure a breath alcohol level and are often used to measure intoxication.

The devices are typically relatively expensive and are designed to be re-used multiple times. However the breath of a subject is a warm, moist environment and therefore often contains numerous microbes, such as bacteria and viruses. It is therefore desirable to allow a sample of exhaled gases to enter the device, but to prevent microbes associated with the exhaled gases to reach the device to prevent a microbiological build-up in the device. Such a build-up of microbiological matter may pose an infection risk for subsequent users of the device. It is also desirable to prevent particulate matter that may also be associated with exhaled gases to enter the device and thereby impair the sensor components of the device. WO 2011/143289 A2 discloses a mouthpiece having a filter forming a cylindrical annular channel to receive a cylindrical tube.

According to a first aspect of the invention, there is provided a device mouthpiece comprising a cylindrical tube and a filter element, wherein the filter element comprises a frame and a filter medium secured to the frame; the frame includes a cylindrical body portion at one end of which is located an annular channel defined by a portion of the cylindrical body portion which has an outwardly facing surface, and an outer wall which is spaced from the inner wall and has an inwardly facing surface, wherein the annular channel receives therein an end portion of the cylindrical tube; the outwardly facing surface of the cylindrical body portion carries or defines radially outwardly extending ribs; the cylindrical body portion of the frame is located within the cylindrical tube; the end portion of the cylindrical tube forms a friction fit within the annular channel; wherein the filter element is secured to the cylindrical tube via the friction fit; and wherein the frame is a polymeric frame, and the filter medium is ultrasonically welded to a portion of the internal cylindrical channel defined by the cylindrical body portion such that the weld extends around the entire circumference of the cylindrical channel.

The device mouthpiece of the invention is suitably disposable and may be intended for single patient use. The patient may use the mouthpiece on multiple occasions or they may use it for a single use. The cylindrical tube forms a conduit to direct exhaled gases into a gas analyser and the filter medium allows gases to pass therethrough, but prevents the passage therethrough of micro-biological matter (microbes), saliva and/or particulate material. As the filter medium is intended to capture the microbiological matter, saliva and/or particulate material and is disposable, the risk of infection to subsequent users, each of whom would use a new, unused device mouthpiece is minimised.

The filter element includes a portion which fits over one end of the cylindrical tube. This is in the form of an annular channel defined by the frame which is sized and configured to receive therein an end portion of the cylindrical tube. The filter element includes a substantially cylindrical body portion which in use is located within the cylindrical tube. The substantially cylindrical body portion forms a tight or friction fit within the cylindrical tube.

The annular channel is located at one end of the cylindrical body portion.

The cylindrical tube forms a friction fit within the annular channel. This has the effect of coupling the filter element to the cylindrical tube. In order to form the friction fit more securely, at least one surface of the annular channel may include a plurality of radially extending ribs. Thus, the annular channel defines an inner wall (e.g. a portion of the cylindrical body portion) which has an outwardly facing surface, and an outer wall or skirt which is spaced from the inner wall and has an inwardly facing surface, wherein the cylindrical tube in use is located between the outwardly facing surface of the inner wall and the inwardly facing surface of the outer wall. The outwardly facing surface of the inner wall carries or defines radially outwardly extending ribs and/or the inwardly facing surface of the outer wall may carry or define radially inwardly extending ribs. The ribs are configured to bite into the wall of the cylindrical tube when an end of it is urged into the annular channel and thereby increase the friction between the filter element and the cylindrical tube.

The ribs may be circumferentially spaced around the annular channel.

In order to trap micro-biological matter without restricting unduly airflow through the medium, the filter medium is suitably an electrostatic filter medium. Optionally, the filter medium is a non-woven electrostatic filter medium, such as a non-woven polymeric electrostatic filter medium.

According to the invention, the filter medium is secured to the frame. This may be achieved by locating the filter medium between two spaced apart frame portions (i.e. trapping the filter medium between two portions of the frame) or by fixing or attaching the filter medium to at least a portion of the frame.

By forming a weld between the filter medium and the frame around the entire circumference of the frame, no gaps exist between the filter medium and the frame and as such, the filter medium is able to trap a greater percentage of microbes, saliva and/or particulate matter.

The frame may be formed by injection moulding and may therefore be a polymeric, injection moulded frame. The frame may be formed, for example, from a polyalkylene, such as polypropylene.

In an embodiment of the invention, the frame is a polymeric frame which defines an internal cylindrical channel and the filter medium is ultrasonically welded to a portion of the internal cylindrical channel such that the weld extends around the entire circumference of the cylindrical channel. In the context of the present invention, the portion of the internal cylindrical channel to which the filter medium is welded includes the annular end face of the cylindrical channel.

By securing the filter medium to the frame, the filter medium is able to filter a majority of the exhaled gases passing through the filter element. Adhering the filter medium to the frame provides a better seal between the filter medium and the frame which reduces the volume of unfiltered gases able to pass through the filter element. However, ultrasonically welding the filter medium around the entire circumference of the frame provides a still better seal between the filter medium and the frame which reduces still further the volume of unfiltered gases able to pass through the filter element.

In order to support the filter medium in use, the frame may define an internal cylindrical channel and two or more radial support elements which extend across the channel. The radial support elements may reduce axial deflection of the filter medium in use. By supporting the filter medium in this way, the risk of accidental swallowing of the filter medium is reduced.

As noted above, the device mouthpiece is suitably a disposable component. Accordingly, the cylindrical tube is suitably made from a material that is cheap and easy to produce and which is relatively non-damaging to the environment after disposal. Accordingly, the cylindrical tube may be formed from a fibrous material, such as a compressed fibre material, suitably cardboard. The cylindrical tube may comprise a plurality of layers of the fibrous material in order to provide the desired strength characteristics.

The outer surface of the cylindrical tube may be coated with a polymeric barrier layer. The barrier layer prevents direct contact between the user's lips and the fibrous material of the tube. This eliminates the risk of a chemical reaction between the user and chemicals that may be present in the fibrous material; it enhances the seal between the device mouthpiece and the device with which the mouthpiece is being used; and it prevents or reduces the risk of the user's lips sticking to the fibrous material. The barrier layer may be formed from a polyalkylene, such as polyethylene.

The skilled person will appreciate that the features described and defined in connection with the aspect of the invention and the embodiments thereof may be combined in any combination, regardless of whether the specific combination is expressly mentioned herein. Thus, all such combinations are considered to be made available to the skilled person.

An embodiment of the invention will now be described, by way of example only, with reference to the accompanying drawings in which:
Figure 1 is a cross-section through a cylindrical tube which forms part of the invention;
Figure 2 is a magnified section through a wall of the cylindrical tube shown in Figure 1;
Figure 3 is a perspective view of a filter element which forms part of the invention; and
Figure 4 is a perspective view of the filter element shown in Figure 3 from a different angle.

For the avoidance of doubt, the skilled person will appreciate that in this specification, the terms "up", "down", "front", "rear", "upper", "lower", "width", etc. refer to the orientation of the components as found in the example when installed for normal use as shown in the Figures.

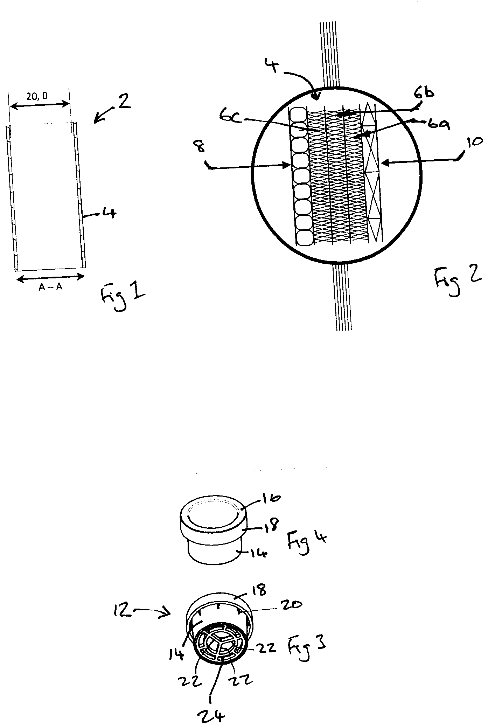

As shown in Figure 1, a cylindrical tube 2 is provided which is of a simple, disposable construction. The tube 2 is defined by a wall 4 and is open at both ends. The wall 4 is shown in more detail in Figure 2, where it can be seen that the wall 4 is a laminate including a core formed from three layers of a Kraft liner paper 6a, 6b, 6c; an outer layer 8 formed from a paper coated with polyethylene; and an inner layer 10 formed from a paper coated with polyvinyl acetate. The layers are adhered together with a polyvinyl acetate adhesive to form the rigid, polymer-coated tube 2. The tube is between 60 and 70mm long and has an internal diameter of 20mm and an outer diameter of 22mm.

Figure 3 shows a filter element 12 including a cylindrical main body portion 14 which is open at both ends. At one end of the main body portion 14 is an outwardly extending top wall 16 (seen in Figure 4) and a radially downwardly extending skirt portion 18, wherein the skirt portion 18 is coaxial with the main body portion 14 and spaced outwardly therefrom to define an annular channel between the main body portion 14 and the skirt 18, the channel being closed at one end by the top wall 16 and open at the opposite end.

The top end of the main body portion 14 (i.e. the end adjacent to the top wall 16) includes a plurality of radially outwardly projecting ribs 20. The ribs 20 are circumferentially spaced around the outer surface of the main body portion 14 and extend axially such that they extend beyond the length of the channel.

At the bottom end of the main body portion 14, there is provided three radially inwardly extending supporting arms 22 which meet at the longitudinal axis of the body portion 14. Also provided at the bottom end of the main body portion 14 is an annular supporting ring 24.

An electrostatic filter medium (not shown) is ultrasonically welded to the bottom end of the main body portion 14, such that the weld extends around the entire circumference of the bottom of the main body portion 14. In other words, the electrostatic filter medium is welded to the downwardly facing peripheral wall of the main body portion 14. The filter medium is supported in place by the radial supporting arms 22 and the supporting ring 24, such that it is not able to enter the cylindrical channel defined by the main body portion 14.

The annular channel defined between the main body portion 14 and the skirt 18 is sized to receive therein the cylindrical tube 2. Thus, the outer diameter of the main body portion 14 is 20mm so that the main body portion 14 fits snugly within the tube 2 and the inner diameter of the skirt 18 is 22.2mm such that an end portion of the tube 2 is able to fit snugly within the channel. The axial ribs 20 engage the internal surface of the tube 2 such that the main body portion 14 forms a tight friction fit within the tube 2 and the end portion of the tube 2 forms a friction fit within the annular channel. This arrangement resists the removal of the filter element 12 from the tube 2.

The ultrasonic weld between the peripheral edge of the filter medium and the entire circumference of the main body portion 14 prevents any exhaled gases from passing between the filter medium and the main body portion 14. Thus, all exhaled gases must pass through the filter medium whereupon microbes and particulate matter entrained within the flow of exhaled gases are trapped by the filter medium.

In use, the main body portion 14 of the filter element 12 is urged into the top end of the tube 2 until the top end of the tube 2 is located snugly within the annular channel. The bottom end of the tube is then located into a receiving portion of a gas analysing device and a user breathes into the device via the combined tube 2 and filter element 12 (together a "device mouthpiece"). The filter medium filters the exhaled gases and prevents contaminants reaching the device. After use, the device mouthpiece is discarded and a fresh mouthpiece is used for the next user.

## Claims

1. A device mouthpiece comprising a cylindrical tube (2) and a filter element (12), wherein the filter element (12) comprises a frame and a filter medium secured to the frame; the frame includes a cylindrical body portion (14) at one end of which is located an annular channel defined by a portion of the cylindrical body portion (14) which has an outwardly facing surface, and an outer wall (18) which is spaced from the inner wall (14) and has an inwardly facing surface, wherein the annular channel receives therein an end portion of the cylindrical tube (2); the outwardly facing surface of the cylindrical body portion (14) carries or defines radially outwardly extending ribs (20); the cylindrical body portion (14) of the frame is located within the cylindrical tube (2); the end portion of the cylindrical tube (2) forms a friction fit within the annular channel; wherein the filter element (12) is secured to the cylindrical tube (2) via the friction fit; and wherein the frame is a polymeric frame, and the filter medium is ultrasonically welded to a portion of the internal cylindrical channel defined by the cylindrical body portion (14) such that the weld extends around the entire circumference of the cylindrical channel.

2. A device mouthpiece according to Claim 1, wherein the inwardly facing surface of the outer wall (18) includes a plurality of radially extending ribs.

3. A device mouthpiece according to Claim 1 or Claim 2, wherein the filter medium is an electrostatic filter medium.

4. A device mouthpiece according to any of Claims 1 to 3, wherein two or more radial support elements (22) extend across the internal cylindrical channel.

5. A device mouthpiece according to any of Claims 1 to 4, wherein the cylindrical tube (2) comprises a body formed from a fibrous material.

6. A device mouthpiece according to Claim 5, wherein the fibrous material is cardboard.

7. A device mouthpiece according to Claim 5 or Claim 6, wherein the outer surface of the cylindrical tube (2) is coated with a polymeric barrier layer.

## Patentansprüche

1. Gerätemundstück, umfassend ein zylindrisches Rohr (2) und ein Filterelement (12), wobei das Filterelement (12) einen Rahmen und ein an dem Rahmen gesichertes Filtermedium umfasst; der Rahmen einen zylindrischen Körperabschnitt (14) beinhaltet, an dessen einem Ende sich ein ringförmiger Kanal befindet, der definiert ist durch einen Abschnitt des zylindrischen Körperabschnitts (14), der eine nach außen weisende Oberfläche aufweist, und eine Außenwand (18), die von der Innenwand (14) beabstandet ist und eine nach innen weisende Oberfläche aufweist, wobei der ringförmige Kanal darin einen Endabschnitt des zylindrischen Rohrs (2) aufnimmt; die nach außen weisende Oberfläche des zylindrischen Körperabschnitts (14) sich radial nach außen erstreckende Rippen (20) trägt oder definiert; sich der zylindrische Körperabschnitt (14) des Rahmens innerhalb des zylindrischen Rohrs (2) befindet; der Endabschnitt des zylindrischen Rohrs (2) eine Reibungspassung innerhalb des ringförmigen Kanals bildet; wobei das Filterelement (12) an dem zylindrischen Rohr (2) mittels der Reibungspassung gesichert ist; und wobei der Rahmen ein polymerer Rahmen ist und das Filtermedium an einen Abschnitt des internen zylindrischen Kanals, der durch den zylindrischen Körperabschnitt (14) definiert ist, ultraschallgeschweißt ist, sodass sich die Schweißung um den gesamten Umfang des zylindrischen Kanals erstreckt.

2. Gerätemundstück nach Anspruch 1, wobei die nach innen gerichtete Oberfläche der Außenwand (18) eine Vielzahl von sich radial erstreckenden Rippen beinhaltet.

3. Gerätemundstück nach Anspruch 1 oder Anspruch 2, wobei das Filtermedium ein elektrostatisches Filtermedium ist.

4. Gerätemundstück nach einem der Ansprüche 1 bis 3, wobei sich zwei oder mehr radiale Stützelemente (22) über den internen zylindrischen Kanal erstrecken.

5. Gerätemundstück nach einem der Ansprüche 1 bis 4, wobei das zylindrische Rohr (2) einen aus einem Fasermaterial gebildeten Körper umfasst.

6. Gerätemundstück nach Anspruch 5, wobei das Fasermaterial Pappe ist.

7. Gerätemundstück nach Anspruch 5 oder Anspruch 6, wobei die Außenfläche des zylindrischen Rohrs (2) mit einer polymeren Barriereschicht beschichtet ist.

## Revendications

1. Une embouchure de dispositif comprenant un tube cylindrique (2) et un élément filtrant (12), dans laquelle l'élément filtrant (12) comprend un cadre et un milieu filtrant fixé au cadre ; le cadre inclut une partie corps cylindrique (14) à une extrémité de laquelle est situé un canal annulaire défini par une partie de la partie corps cylindrique (14) qui a une surface tournée vers l'extérieur et une paroi extérieure (18) qui est espacée de la paroi intérieure (14) et a une surface tournée vers l'intérieur, dans laquelle le canal annulaire y reçoit une partie d'extrémité du tube cylindrique (2) ; la surface tournée vers l'extérieur de la partie corps cylindrique (14) porte ou définit des nervures s'étendant radialement vers l'extérieur (20) ; la partie corps cylindrique (14) du cadre est située à l'intérieur du tube cylindrique (2) ; la partie d'extrémité du tube cylindrique (2) forme un ajustement serré à l'intérieur du canal annulaire ; dans laquelle l'élément filtrant (12) est fixé au tube cylindrique (2) par l'ajustement serré ; et dans laquelle le cadre est un cadre en polymère, et le milieu filtrant est soudé par ultrasons à une partie du canal cylindrique interne définie par la partie corps cylindrique (14) de sorte que la soudure s'étend tout autour de la circonférence du canal cylindrique.

2. Une embouchure de dispositif selon la revendication 1, dans laquelle la surface tournée vers l'intérieur de la paroi extérieure (18) inclut une pluralité de nervures s'étendant radialement.

3. Une embouchure de dispositif selon la revendication 1 ou la revendication 2, dans laquelle le milieu filtrant est un milieu filtrant électrostatique.

4. Une embouchure de dispositif selon l'une quelconque des revendications 1 à 3, dans laquelle deux éléments de support radiaux (22) ou plus s'étendent sur tout le canal cylindrique interne.

5. Une embouchure de dispositif selon l'une quelconque des revendications 1 à 4, dans laquelle le tube cylindrique (2) comprend un corps formé d'un matériau fibreux.

6. Une embouchure de dispositif selon la revendication 5, dans laquelle le matériau fibreux est du carton.

7. Une embouchure de dispositif selon la revendication 5 ou la revendication 6, dans laquelle la surface extérieure du tube cylindrique (2) est revêtue d'une couche barrière polymère.
